# EUROPÄISCHE PATENTANMELDUNG

(11) **EP 3 795 192 A1**
(43) Veröffentlichungstag der Anmeldung: **24.03.2021**
(21) Anmeldenummer: 20197488.8
(22) Anmeldetag: 22.09.2020
(51) Int. Cl.: A61M 1/36, A61M 1/16

(54) **DICHTIGKEITSPRÜFVERFAHREN FÜR EINE APPARATUR ZUR TEMPERIERUNG VON BLUT**

(30) Priorität: 23.09.2019 DE 102019125524
(71) Anmelder: Lauda Dr. R. Wobser GmbH & Co. KG, 97922 Lauda-Königshofen (DE)
(72) Erfinder: Firmbach, Peter, 97947 Grünsfeld (DE)
(74) Vertreter: Zimmermann & Partner Patentanwälte mbB

(57) **Zusammenfassung**

Ein Hypo-/Hyperthermiegerät und ein Dichtigkeitsprüfverfahren für einen Temperierkreislauf für eine Apparatur zur Temperierung von Blut werden hierin beschrieben. Das Hypo-/Hyperthermiegerät weist eine Vorlaufleitung mit einem Vorlaufanschluss zum Anschluss an die Apparatur und einem in der Vorlaufleitung angeordneten Vorlaufventil auf. Ferner weist das Hypo-/Hyperthermiegerät eine Rücklaufleitung mit einem Rücklaufanschluss zum Anschluss an die Apparatur und einem in der Rücklaufleitung angeordneten Rücklaufventil. Das Hypo-/Hyperthermiegerät weist eine Pumpe, welche zwischen der Rücklaufleitung und der Vorlaufleitung zur Förderung eines flüssigen Temperiermediums in einer Förderrichtung von der Rücklaufleitung zu der Vorlaufleitung angeordnet ist; und ein Druckmessgerät zum Messen eines Drucks in der Vorlaufleitung oder in der Rücklaufleitung auf. Weiterhin wird ein Dichtigkeitsprüfverfahren für einen Temperierkreislauf für eine Apparatur zur Temperierung von Blut beschrieben.

## Beschreibung

### Gebiet der Erfindung

Die Erfindung betrifft Hypo-/Hyperthermiegeräte und Dichtigkeitsprüfverfahren für einen Temperierkreislauf für eine Apparatur zur Temperierung von Blut.

### Stand der Technik

Aus dem Stand der Technik sind Apparaturen zur Ausbildung eines Temperierkreislaufs zur Temperierung von Blut bekannt, insbesondere Oxygenatoren. Oxygenatoren sind dazu eingerichtet einen Blutkreislauf, einen Sauerstoffkreislauf und einen Wasserkreislauf auszubilden, wobei der Oxygenator jeweils einen Teil des Kreislaufs ausbildet. Um einen Wasserkreislauf zu ermöglichen, ist eine Pumpe an den Oxygenator angeschlossen, die dazu eingerichtet ist Wasser durch den Wasserkreislauf zu fördern. Häufig ist ein Hypo-/Hyperthermiegerät mit dem Oxygenator verbunden. Das mit dem Oxygenator verbundene Hypo-/Hyperthermiegerät bildet zusammen mit dem Oxygenator einen Wasserkreislauf aus, wobei das Hypo-/Hyperthermiegerät typischerweise die Pumpe aufweist und den Wasserkreislauf temperiert. Der Oxygenator weist einen Wärmetauscher auf, der eine Wärmeübertragung zwischen dem Wasserkreislauf und dem Blutkreislauf ermöglicht.

Der in dem Wasserkreislauf herrschende Druck unterscheidet sich typischerweise von dem in dem Blutkreislauf herrschenden Druck, wobei sowohl Überdrucke als auch Unterdrücke vorliegen können. Sollte der Oxygenator, beispielsweise der Wärmetauscher zwischen dem Blutkreislauf und dem Wasserkreislauf, oder eine andere mit dem Blutkreislauf oder dem Wasserkreislauf verbundene Komponente, undicht sein, kann je nach Druckverhältnis (Überdruck oder Unterdruck) entweder Wasser in den Blutkreislauf oder Blut in den Wasserkreislauf gelangen.

Aus dem Stand der Technik sind Versuche unternommen worden mittels einem aufwendigen und nicht aussagekräftigen Drucklufttest den Oxygenator vor Verwendung auf Dichtigkeit zu prüfen. Die mittels Druckluft erzeugten Drücke spiegeln nicht die typischerweise vorherrschenden, realen Druckverhältnisse wieder, die sich im Gebrauch einstellen oder eingestellt werden. Die typischerweise im Gebrauch vorherrschenden Druckverhältnisse sind je nach Temperieraufgabe, Schlauchlänge, Durchflussmengen und anderer Faktoren unterschiedlich. Weiterhin besteht die Gefahr, dass bei dem Drucktest der Oxygenator beschädigt wird.

Ein weiterer aus dem Stand der Technik bekannter Test zum Prüfen der Dichtigkeit besteht darin, das durch den Oxygenator fließende Wasser auf rötliche Verfärbungen durch eingedrungenes Blut (sichtbar an transparenten Wasserschläuchen oder Wasserleitungen) hin zu untersuchen. Dieser Test ist an sich bereits aus mehreren Gründen begrenzt geeignet oder sogar ungeeignet, beispielsweise wenn ein höherer Druck im Wasserkreislauf als im Blutkreislauf vorherrscht. Beispielsweise muss in regelmäßigen Abständen eine Prüfung durch eine Person erfolgen. Zusätzlich werden in vielen Anwendungen keine transparenten, sondern beispielsweise blaue Wasserschläuche oder Wasserleitungen verwendet, wodurch eine Verfärbung nicht sichtbar wird.

### Offenbarung der Erfindung

Aufgabe der Erfindung ist es, einen gegenüber dem Stand der Technik verbessertes Hypo-/Hyperthermiegerät und ein verbessertes Dichtigkeitsprüfverfahren für einen Temperierkreislauf für eine Apparatur zur Temperierung von Blut bereitzustellen.

Die Aufgabe wird z. B. gelöst durch einen Hypo-/Hyperthermiegerät gemäß Anspruch 1 oder durch ein Verfahren gemäß dem nebengeordneten Anspruch. Weiterbildungen des Verfahrens oder der Vorrichtung sind in den abhängigen Ansprüchen angegeben.

Ein erster Aspekt betrifft ein Hypo-/Hyperthermiegerät zur Verbindung mit einer Apparatur zur Ausbildung eines Temperierkreislaufs zur Temperierung von Blut. Insbesondere ist die Apparatur ein Oxygenator oder ein Plegiekreislauf. Das Hypo-/Hyperthermiegerät weist eine Vorlaufleitung mit einem Vorlaufanschluss zum Anschluss an die Apparatur und einem in der Vorlaufleitung angeordneten Vorlaufventil auf. Ferner weist das Hypo-/Hyperthermiegerät eine Rücklaufleitung mit einem Rücklaufanschluss zum Anschluss an die Apparatur und einem in der Rücklaufleitung angeordneten Rücklaufventil. Das Hypo-/Hyperthermiegerät weist eine Pumpe, welche zwischen der Rücklaufleitung und der Vorlaufleitung zur Förderung eines flüssigen Temperiermediums in einer Förderrichtung von der Rücklaufleitung zu der Vorlaufleitung angeordnet ist, und ein Druckmessgerät zum Messen eines Drucks in der Vorlaufleitung oder in der Rücklaufleitung auf.

Das Hypo-/Hyperthermiegerät kann mit der Apparatur zur Ausbildung des Temperierkreislaufs, insbesondere dem Oxygenator oder dem Plegiekreislauf, verbunden sein, wobei der Vorlaufanschluss und der Rücklaufanschluss des Hypo-/Hyperthermiegerät jeweils mittels eines Schlauchs oder einer Leitung mit einem Anschluss mit der Apparatur verbunden sein können. Der Temperierkreislauf wird durch das Hypo-/Hyperthermiegerät, der Apparatur, und optional den Schläuchen oder Leitungen, gebildet.

Das im Temperierkreislauf verwendete flüssige Temperiermedium ist typischerweise Wasser, beispielsweise eine isotonische Kochsalzlösung oder eine andere isotonische oder nicht-isotonische Lösung.

Das Druckmessgerät kann in der Vorlaufleitung zwischen dem Vorlaufventil und dem Vorlaufanschluss oder in der Rücklaufleitung zwischen dem Rücklaufanschluss und dem Rücklaufventil angeordnet sein.

Der an den Rücklaufanschluss angeschlossene Schlauch oder Leitung kann in manchen Ausführungsformen als Teil der Rücklaufleitung betrachtet werden. Der an den Vorlaufanschluss angeschlossene Schlauch oder Leitung kann in manchen Ausführungsformen als Teil der Vorlaufleitung betrachtet werden. Gemäß einer Ausführungsform kann das Druckmessgerät in dem an den Rücklaufanschluss angeschlossenen Schlauch oder Leitung angeordnet sein. Gemäß einer Ausführungsform kann das Druckmessgerät in dem an den Vorlaufanschluss angeschlossenen Schlauch oder Leitung angeordnet sein.

Das Hypo-/Hyperthermiegerät gemäß der vorliegenden Offenbarung ermöglicht es eine verbesserte Dichtigkeitsprüfung der mit dem Hypo-/Hyperthermiegerät verbindbaren Apparatur durchzuführen, wie nachfolgend näher beschrieben wird.

Das Hypo-/Hyperthermiegerät kann eine Kontrolleinheit aufweisen, welche mit dem Vorlaufventil, dem Rücklaufventil und dem Druckmessgerät verbunden ist. Die Kontrolleinheit kann eingerichtet sein eine Dichtigkeitsprüfung durchzuführen. Die Dichtigkeitsprüfung umfasst ein Schließen des Rücklaufventils. Falls die Pumpe bei Schließen des Rücklaufventils nicht im Betrieb ist, betreibt die Kontrolleinheit die Pumpe. Falls die Pumpe bei Schließen des Rücklaufventils im Betrieb ist, wird die Pumpe unverändert von der Kontrolleinheit betrieben. Das Betreiben der Pumpe ermöglicht es einen Überdruck zwischen der Pumpe und dem Rücklaufventil aufzubauen.

In einer Ausführungsform ist das Druckmessgerät dazu eingerichtet, nach Schließen des Rücklaufventils in regelmäßigen Abständen, beispielsweise mindestens alle 120 Sekunden, mindestens alle 300 Sekunden oder höchstens alle 600 Sekunden oder höchstens alle 360 Sekunden, den Druck zu messen.

Die Kontrolleinheit kann dazu eingerichtet sein das Vorlaufventil zu schließen. Insbesondere kann die Kontrolleinheit dazu eingerichtet sein das Vorlaufventil erst dann zu schließen, wenn von dem Druckmessgerät ein zumindest im Wesentlichen konstanter Druck gemessen wird. In einer beispielhaften Ausführungsform kann ein im Wesentlichen konstanter Druck bedeuten, dass bei zwei hintereinander folgenden Druckmessungen, insbesondere bei einem zeitlichen Abstand von ca. 10 Sekunden, eine Abweichung weniger als 3 % beträgt. Bei Überschreiten einer vorgegebenen Zeit, beispielsweise 30 Sekunden, ohne dass ein im Wesentlichen konstanter Druck gemessen wird, kann die Kontrolleinheit dazu konfiguriert sein eine Warnmitteilung auszugeben. "Zumindest im Wesentlichen konstanter Druck" kann eine Druckänderung von weniger als 5%, weniger als 3% bedeuten oder maximal 1% bedeuten. Die verglichenen Druckmessungen können einen zeitlichen Abstand von mindestens 3 Sekunden, mindestens 5 Sekunden, oder höchstens 10 Sekunden oder höchstens 20 Sekunden aufweisen.

Ferner kann die Dichtigkeitsprüfung, nach Schließen des Vorlaufventils, ein Messen eines ersten Kontroll-Drucks zwischen dem Vorlaufventil und dem Rücklaufventil mittels des Druckmessgeräts umfassen.

Die Kontrolleinheit kann dazu konfiguriert sein, einen zweiten Kontroll-Druck in einem vorbestimmten Zeitabstand nach dem ersten Kontroll-Druck zu messen. Zwischen der Messung des ersten Kontroll-Drucks und dem zweiten Kontrolldruck sind das Vorlaufventil und das Rücklaufventil (weiterhin) geschlossen. Beispielsweise kann die Kontrolleinheit dazu konfiguriert sein, einen zweiten Kontroll-Druck etwa 20 Sekunden nach dem ersten Kontroll-Druck zu messen. Nach der Messung des zweiten Kontroll-Drucks können das Vorlaufventil und das Rücklaufventil wieder geöffnet werden. Alternativ, falls die Dichtigkeitsprüfung die Messung von mehr als zwei Kontroll-Drücken umfasst, können das Vorlaufventil und das Rücklaufventil wieder geöffnet werden nach der Messung eines letzten Kontroll-Drucks. Die Kontrolleinheit kann dazu konfiguriert sein, eine Druckänderung zwischen dem ersten Kontroll-Druck und dem zweiten Kontroll-Druck zu ermitteln.

Ferner kann die Kontrolleinheit dazu konfiguriert sein, eine Warnmitteilung auszugeben, falls die zwischen dem ersten Kontroll-Druck und dem zweiten Kontroll-Druck auftretende Druckänderung oberhalb eines definierten Grenzwertes liegt. Der Grenzwert kann abhängig von den Eigenschaften von dem Oxygenator oder dem Hypo-/Hyperthermiegerät, insbesondere einem Volumenstrom der Pumpe und einem Leitungsvolumina, sein. Typischerweise beträgt das Leitungsvolumen des Temperierkreislaufs mindestens 3 Liter oder nicht mehr als 6 Liter. Typischerweise ist die Pumpe dazu eingerichtet einen maximalen Volumenstrom von 10 Liter pro Minute zu fördern. Gemäß einer Ausführungsform beträgt der Grenzwert für die Druckänderung mindestens 0,001 bar/s, bevorzugt mindestens 0,005 bar/s und noch bevorzugter mindestens 0,01 bar/s.

Gemäß einer Ausführungsform kann die Kontrolleinheit konfiguriert sein zum automatischen Initiieren der Dichtigkeitsprüfung. Automatisches Initiieren bedeutet hierbei, dass die vorstehend genannten Schritte der Dichtigkeitsprüfung von der Kontrolleinheit ausgeführt werden, ohne dass es für einen Benutzer erforderlich ist tätig zu werden oder ohne dass es für einen Benutzer erforderlich ist, einen der vorstehend genannten Schritte der Dichtigkeitsprüfung eigenständig auszuführen. Das automatische Initiieren kann weiterhin umfassen, nach einem Abschluss der Dichtigkeitsprüfung, das Vorlaufventil und das Rücklaufventil zu öffnen, wodurch die Förderung des flüssigen Temperiermediums durch den Temperierkreislauf gewährleistet sein kann. In anderen Worten ausgedrückt, kann die Kontrolleinheit konfiguriert sein, nach Abschluss der Dichtigkeitsprüfung einen Normalbetrieb für den Temperierkreislauf zu ermöglichen. Gemäß einer Ausführungsform dauert die Durchführung der Dichtigkeitsprüfung mindestens 1 Sekunde, bevorzugt mindestens 10 Sekunden, und noch bevorzugter mindestens 20 Sekunden oder höchstens 60 Sekunden, bevorzugt höchstens 40 Sekunden, und noch bevorzugter höchstens 20 Sekunden.

Bei einer Ausführungsform kann die Kontrolleinheit dazu eingerichtet sein, nach einer Inbetriebnahme der Kontrolleinheit oder nach einer Inbetriebnahme der Pumpe die Dichtigkeitsprüfung durchzuführen. Hierdurch kann bei einer Inbetriebnahme der Pumpe ermittelt werden, ob die Apparatur, insbesondere der Temperierkreislauf oder ein Wärmetauscher der Apparatur, zwischen einem Blutkreislauf und dem Temperierkreislauf, dicht ist.

Die Kontrolleinheit kann ferner konfiguriert sein, die Dichtigkeitsprüfung in vorbestimmten Zeitintervallen durchzuführen. Beispielsweise kann eine Dichtigkeitsprüfung in Zeitintervallen von mindestens 2 Minuten, mindestens 5 Minuten oder höchstens 30 Minuten oder höchstens 20 Minuten durchgeführt werden. Alternativ kann die Dichtigkeitsprüfung durch einen Benutzer initiiert werden. Die Kontrolleinheit kann dazu eingerichtet sein, einen Hinweis auszugeben dass das vorbestimmte Zeitintervall abgelaufen ist, und erst dann die Dichtigkeitsprüfung durchführen, wenn eine Freigabe zur Durchführung der Dichtigkeitsprüfung, beispielsweise eine Bestätigung durch Fachpersonal, erfolgt ist.

Das Durchführen der Dichtigkeitsprüfung kann zu bestimmten Zeitpunkten unerwünscht oder sogar unvorteilhaft sein. Wenn beispielsweise eine erhebliche Temperierungsanforderung an den Temperierkreislauf benötigt oder erwünscht ist, insbesondere falls eine Temperatur des Blutkreislaufs erheblich höher oder niedriger als ein Sollwert ist, kann das Durchführen der Dichtigkeitsprüfung unvorteilhaft sein, da der Temperierkreislauf zeitweilig unterbrochen wird und eine Temperierung des Blutkreislaufs zeitweilig nicht möglich ist. Die Förderung anderer Kreisläufe der mit dem Hypo-/Hyperthermiegerät verbindbaren Apparatur, insbesondere der Oxygenator oder der Plegiekreislauf, können von der Dichtigkeitsprüfung unbeeinflusst sein. Insbesondere kann die Förderung eines Blutkreislaufs unbeeinflusst von der Dichtigkeitsprüfung sein, wobei die Temperierung des Blutkreislaufs zeitweilig nur eingeschränkt während der Dichtigkeitsprüfung stattfinden kann.

Gemäß einer Ausführungsform kann die Kontrolleinheit dazu konfiguriert sein, vor einem Durchführen der Dichtigkeitsprüfung ein Abfragen einer Temperierungsanforderung durchzuführen, insbesondere eine Abfrage von dem Hypo-/Hyperthermiegerät. Die Temperierungsanforderung kann beispielsweise einer angeforderten relativen Temperaturänderung entsprechen oder einer angeforderten thermischen Leistung. Die Kontrolleinheit kann dazu eingerichtet sein die Temperierungsanforderung mit einem definierten Schwellenwert zu vergleichen und die Dichtigkeitsprüfung nur dann durchzuführen, falls die Temperierungsanforderung den Schwellenwert nicht überschreitet. Gemäß dieser Ausführungsform wird die Dichtigkeitsprüfung zu Zeitpunkten durchgeführt in denen die Temperierungsanforderungen lediglich gering sind, bzw. bei denen die Temperatur des Blutkreislaufs lediglich geringfügig oder gar nicht von einem Sollwert abweicht. Eine weitere Möglichkeit ist, bei der Temperierungsanforderung abzufragen, ob zu dem jeweiligen Zeitpunkt die Temperatur des Patienten verändert, also erhöht oder erniedrigt, werden soll. Die Kontrolleinheit kann dazu eingerichtet sein die Dichtigkeitsprüfung sofort abzubrechen, falls während der Dichtigkeitsprüfung die Temperierungsanforderung auftritt und insbesondere die Temperierungsanforderung den Schwellenwert überschreitet. Ein sofortiges Abbrechen kann ein Abbrechen innerhalb von höchstens 2 Sekunden oder von höchstens 5 Sekunden bedeuten.

In einer Ausführungsform ist das Hypo-/Hyperthermiegerät dazu geeignet oder dazu zugelassen, in der Temperierung von Patienten eingesetzt zu werden. In einer Ausführungsform ist das Hypo-/Hyperthermiegerät desinfizierbar. In einer Ausführungsform umfasst das Hypo-/Hyperthermiegerät eine redundant ausgelegte Temperaturregelung, oder eine redundante Überhitzungs-Abschaltung, oder eine redundante Pumpenüberwachung bezüglich Leistung, Drehzahl oder Stromaufnahme. In einer Ausführungsform ist die Dichtigkeitsprüfung dazu geeignet Leckagen von bis zu 0,01 mL/s, bevorzugt von bis zu 0,05 mL/s, und noch bevorzugter von bis zu 0,1 mL/s zu detektieren. In einer Ausführungsform ist die Dichtigkeitsprüfung dazu geeignet Druckänderungen von mindestens 0,001 bar/s, bevorzugt mindestens 0,005 bar/s und noch bevorzugter mindestens 0,01 bar/s zu detektieren.

Vorteilhafterweise ermöglicht das Hypo-/Hyperthermiegerät gemäß Ausführungsformen der vorliegenden Offenbarung die automatische oder regelmäßige Überprüfung der Dichtigkeit der Apparatur, insbesondere dem Temperierkreislaufs, oder dem Wärmetauscher zwischen dem Blutkreislauf und dem Temperierkreislauf, ohne dass Fachpersonal die Dichtigkeitsprüfung eigenständig ausführen muss.

Die für die Dichtigkeitsprüfung zusätzlich notwendigen Komponenten sind Komponenten des Hypo-/Hyperthermiegeräts gemäß Ausführungsformen. Hierdurch wird die Dichtigkeitsprüfung mit dem Hypo-/Hyperthermiegerät durch Verbindung mit der Apparatur, insbesondere auch einer bereits vorhandenen oder aus dem Stand der Technik bekannten Apparatur, ermöglicht, ohne dass zusätzliche Komponenten montiert werden müssen oder Veränderungen an der Apparatur, oder angeschlossener Schläuche oder Leitungen, vorgenommen werden müssen. Weiterhin wird ein kompakter Aufbau ermöglicht, da alle zusätzlich für die Dichtigkeitsprüfung benötigten Komponenten in dem Hypo-/Hyperthermiegerät enthalten sein können.

Vorteilhafterweise ermöglicht das Hypo-/Hyperthermiegerät gemäß Ausführungsformen der vorliegenden Offenbarung die Identifizierung einer Leckage der Apparatur, insbesondere einem Oxygenator oder einem Plegiekreislauf, wodurch die Sicherheit von Patienten erhöht werden kann, in dem verhindert wird, dass dessen Blut nicht durch das flüssige Temperiermedium aus dem Temperierkreislauf verdünnt und möglicherweise verunreinigt wird oder in dem verhindert wird, dass dessen Blut aus dem Blutkreislauf abfließt.

Vorteilhafterweise ermöglicht das Hypo-/Hyperthermiegerät gemäß Ausführungsformen der vorliegenden Offenbarung die Überprüfung der Dichtigkeit des Temperierkreislaufs sowohl bei einer Inbetriebnahme des Hypo-/Hyperthermiegeräts, insbesondere bei einer Inbetriebnahme der Kontrolleinheit oder nach Inbetriebnahme der Pumpe, als auch während einem laufenden Betrieb, d.h. ohne die Förderung eines Blutkreislaufs zu unterbrechen, und bei lediglich kurzzeitiger Unterbrechung der Temperierung von Blut.

Typische Vorteile von Ausführungsformen sind die Durchführung der Dichtigkeitsprüfung bei realen Druckverhältnissen, die sich im Gebrauch einstellen oder eingestellt werden. Dies ermöglicht eine wesentlich zuverlässigere Dichtigkeitsprüfung und vermindert oder beseitigt die Gefahr einer Beschädigung der Apparatur.

Ein weiterer Aspekt betrifft ein Dichtigkeitsprüfverfahren für einen Temperierkreislauf für eine Apparatur zur Temperierung von Blut, insbesondere einem Oxygenator oder einem Plegiekreislauf. Das Verfahren wird mittels eines Hypo-/Hyperthermiegerät nach einem der vorhergehenden Ausführungsformen durchgeführt. Insbesondere kann das Dichtigkeitsprüfverfahren die Durchführung während einer akuten Temperierung von Blut eines Patienten umfassen.

Das Dichtigkeitsprüfverfahren kann eine Dichtigkeitsprüfung umfassen mit Schließen des Rücklaufventils; Betreiben der Pumpe, um zwischen der Pumpe und dem Rücklaufventil einen Überdruck aufzubauen; Schließen des Vorlaufventils; und Messen eines ersten Kontroll-Drucks zwischen dem Vorlaufventil und dem Rücklaufventil mittels des Druckmessgeräts.

In einer Ausführungsform wird das Vorlaufventil erst dann geschlossen, wenn von dem Druckmessgerät ein zumindest im Wesentlichen konstanter Druck gemessen wird. Dies ist eine Möglichkeit, um zu erreichen, dass nicht andere als durch Leckage hervorgerufene Druckverluste die Messung verfälschen können. Bei Überschreiten einer vorgegebenen Zeit, beispielsweise 30 Sekunden, ohne dass ein im Wesentlichen konstanter Druck gemessen wird, kann eine Warnmitteilung ausgegeben werden.

Das Dichtigkeitsprüfverfahren kann ferner umfassen: Messen eines zweiten Kontroll-Drucks in einem vorbestimmten Zeitabstand nach dem ersten Kontroll-Druck; und Ausgeben einer Warnmitteilung, falls eine zwischen dem ersten Kontroll-Druck und dem zweiten Kontroll-Druck auftretende Druckänderung oberhalb eines definierten Grenzwertes liegt.

Das Dichtigkeitsprüfverfahren, kann vor einem Durchführen der Dichtigkeitsprüfung, folgendes umfassen: Abfragen einer Temperierungsanforderung und Vergleichen der Temperierungsanforderung mit einem Schwellenwert; und Durchführen der Dichtigkeitsprüfung nur dann, falls die Temperierungsanforderung den Schwellenwert nicht überschreitet.

### Kurze Beschreibung der Zeichnungen

Weitere Vorteile und Merkmale bevorzugter Ausführungsformen der Erfindung werden nachfolgend anhand der beiliegenden Zeichnungen erläutert, wobei die Figuren zeigen:

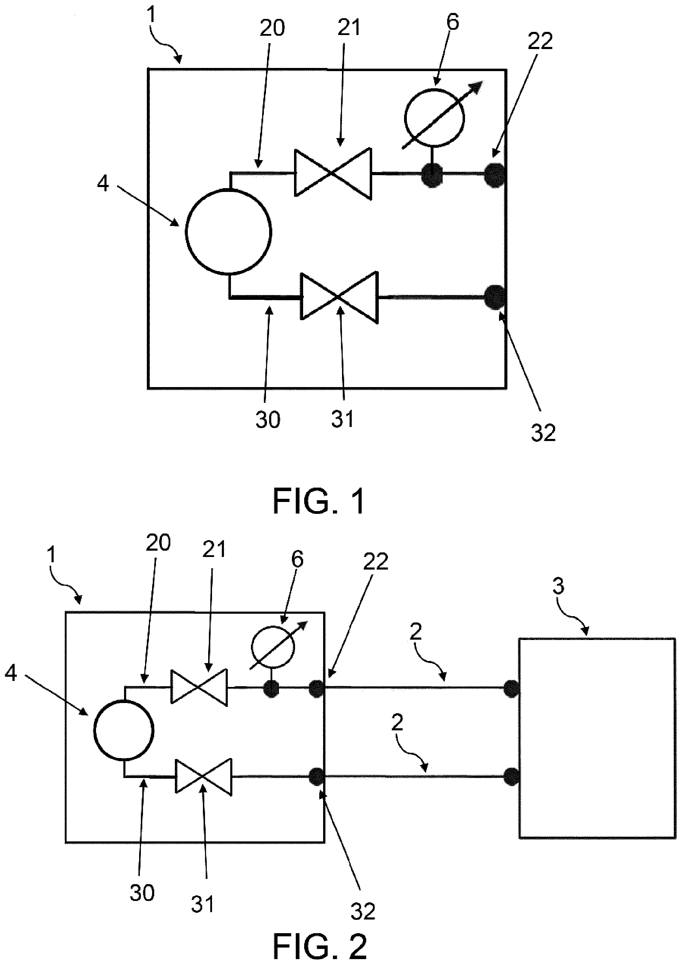

- Fig. 1: ist eine schematische Schnittansicht des Hypo-/Hyperthermiegeräts gemäß einer Ausführungsform der Erfindung;
- Fig.: 2 ist eine schematische Schnittansicht des Hypo-/Hyperthermiegeräts gemäß einer Ausführungsform der Erfindung verbunden mit einer Apparatur;
- Fig.: 3 ist eine schematische Darstellung des Dichtigkeitsprüfverfahrens gemäß einer Ausführungsform der Erfindung; und
- Fig.: 4 ist eine schematische Darstellung des Dichtigkeitsprüfverfahrens gemäß einer Ausführungsform der Erfindung.

### Beschreibung der in den Figuren gezeigten Ausführungsbeispiele

Nachfolgend werden typische Ausführungsformen anhand der Figuren beschrieben, wobei die Erfindung nicht auf die Ausführungsbeispiele beschränkt ist, vielmehr wird der Umfang der Erfindung durch die Ansprüche bestimmt.

In der Fig. 1 ist ein Hypo-/Hyperthermiegerät 1 zur Verbindung mit einer Apparatur, insbesondere einem Oxygenator oder einem Plegiekreislauf, zur Ausbildung eines Temperierkreislaufs zur Temperierung von Blut, gezeigt.

Das Hypo-/Hyperthermiegerät 1 umfasst eine Vorlaufleitung 20 mit einem Vorlaufanschluss 22 und einem in der Vorlaufleitung 20 angeordneten Vorlaufventil 21. Der Vorlaufanschluss 22 kann an die Apparatur 3 angeschlossen sein.

Das Hypo-/Hyperthermiegerät 1 umfasst eine Rücklaufleitung 30 mit einem Rücklaufanschluss 32 und einem in der Rücklaufleitung 30 angeordneten Rücklaufventil 31. Der Rücklaufanschluss 32 kann an die Apparatur 3 angeschlossen sein.

Ferner weist das Hypo-/Hyperthermiegerät 1 eine Pumpe 4 auf, die mit der Rücklaufleitung 30 und der Vorlaufleitung 20 verbunden ist. Die Pumpe 4 ist zwischen der Rücklaufleitung 30 und der Vorlaufleitung 20 angeordnet zur Förderung eines flüssigen Temperiermediums in einer Förderrichtung von der Rücklaufleitung 30 zu der Vorlaufleitung 20. Das im Temperierkreislauf verwendete flüssige Temperiermedium ist typischerweise Wasser.

Das Hypo-/Hyperthermiegerät 1 weist ein Druckmessgerät 6 zum Messen eines Drucks in der Vorlaufleitung 20 oder in der Rücklaufleitung 30 auf. In Figur 1 ist das Druckmessgerät 6 in der Vorlaufleitung 20 zwischen dem Vorlaufventil 21 und dem Vorlaufanschluss 22 angeordnet. Das Druckmessgerät 6 kann alternativ in der Rücklaufleitung 30 zwischen dem Rücklaufanschluss 32 und dem Rücklaufventil 31 angeordnet sein.

Figur 2 zeigt das Hypo-/Hyperthermiegerät 1 verbunden mit der Apparatur 3. In Figur 2 sind der Vorlaufanschluss 22 und der Rücklaufanschluss 32 mittels Schläuchen 2 an die Apparatur 3 angeschlossen.

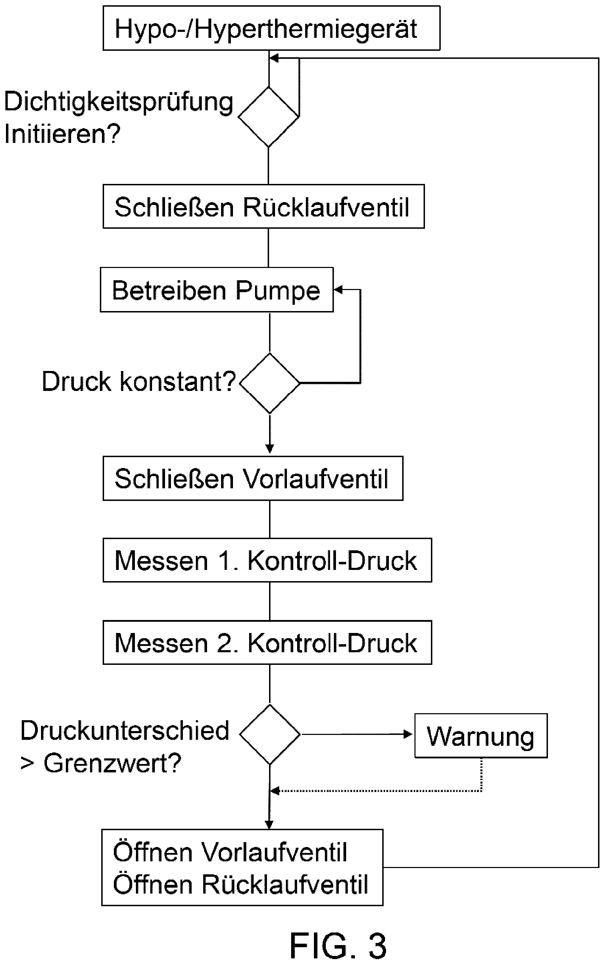

In der Fig. 3 ist ein Dichtigkeitsprüfverfahren für einen Temperierkreislauf für eine Apparatur zur Temperierung von Blut, insbesondere einem Oxygenator oder einem Plegiekreislauf, mittels dem Hypo-/Hyperthermiegerät 1 nach einer der vorhergehenden Ausführungsformen, schematisch dargestellt. Das Dichtigkeitsprüfverfahren umfasst ein automatisches Initiieren der Dichtigkeitsprüfung. Beispielsweise kann das Dichtigkeitsprüfverfahren in vorbestimmten Zeitintervallen wiederholt durchgeführt werden, bei der hier dargestellten Ausführungsform alle 15 Minuten, wobei andere Intervallzeiten möglich sind.

Das Dichtigkeitsprüfverfahren umfasst eine Dichtigkeitsprüfung mit Schließen des Rücklaufventils 31 mittels der Kontrolleinheit und Betreiben der Pumpe 4, um zwischen der Pumpe 4 und dem Rücklaufventil 31 einen Überdruck aufzubauen. Die Dichtigkeitsprüfung kann die Messung des Drucks in regelmäßigen Abständen mittels dem Druckmessgerät 6 umfassen. Falls ein im Wesentlichen konstanter Druck gemessen wird, umfasst die Dichtigkeitsprüfung das Schließen des Vorlaufventils 21 mittels der Kontrolleinheit. Falls kein im Wesentlichen konstanter Druck gemessen wird, kann die Pumpe 4 so lange weiter betrieben werden, bis ein im Wesentlichen konstanter Druck gemessen wird. Bei Überschreiten einer vorgegebenen Zeit, beispielsweise 30 Sekunden, ohne dass ein im Wesentlichen konstanter Druck gemessen wird, kann eine Warnmitteilung ausgegeben werden.

Die Dichtigkeitsprüfung umfasst ferner das Messen eines ersten Kontroll-Drucks zwischen dem Vorlaufventil 21 und dem Rücklaufventil 31 mittels des Druckmessgeräts 6; und das Messen eines zweiten Kontroll-Drucks in einem vorbestimmten Zeitabstand nach dem ersten Kontroll-Druck.

Die Kontrolleinheit kann die auftretende Druckänderung zwischen dem ersten Kontroll-Druck und dem zweiten Kontroll-Drucks ermitteln. Falls die auftretende Druckänderung oberhalb eines definierten Grenzwertes liegt, kann die Kontrolleinheit eine Warnmitteilung ausgeben. Ferner kann, nach Messen des ersten und zweiten Kontroll-Drucks, die Kontrolleinheit das Vorlaufventil 21 und das Rücklaufventil 31 öffnen, optional auch falls die auftretende Druckänderung oberhalb eines definierten Grenzwertes liegt. Hierdurch kann die Förderung des flüssigen Temperiermediums durch den Temperierkreislauf gewährleistet werden. Die Dichtigkeitsprüfung kann nach Ablauf des vorbestimmten Zeitintervalls erneut durchgeführt werden.

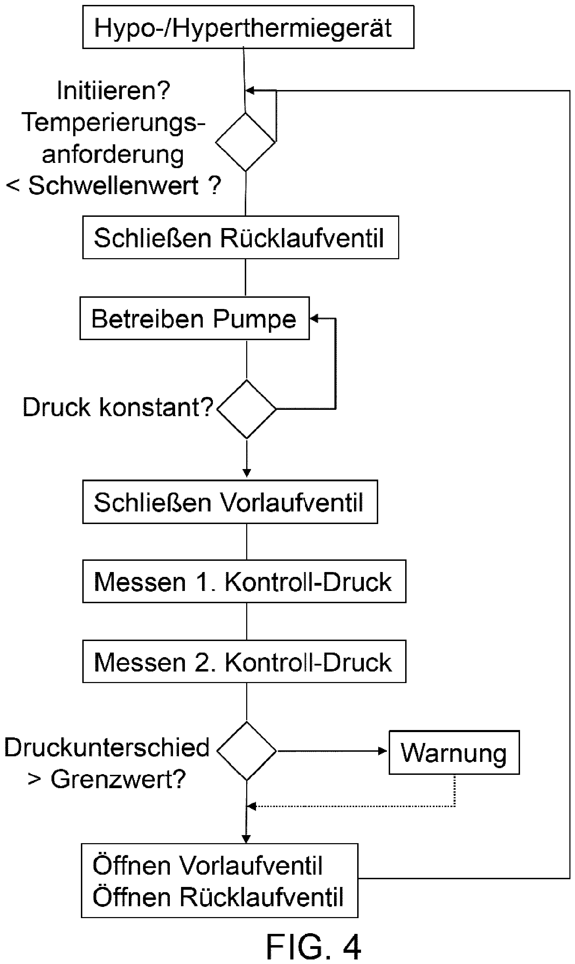

In der Fig. 4 ist ein Dichtigkeitsprüfverfahren gemäß einer Ausführungsform schematisch dargestellt. Zusätzlich zu dem in Figur 3 dargestellten Dichtigkeitsprüfverfahren erfolgt bei der in der Figur 4 dargestellten Ausführungsform vor einem Durchführen der Dichtigkeitsprüfung ein Abfragen einer Temperierungsanforderung mittels der Kontrolleinheit. Ferner erfolgt mittels der Kontrolleinheit ein Vergleichen der Temperierungsanforderung mit einem Schwellenwert. Die nachfolgenden, in Figur 4 dargestellten, Schritte der Dichtigkeitsprüfung werden nur dann durchgeführt, falls die Temperierungsanforderung den Schwellenwert nicht überschreitet.

Falls während der Dichtigkeitsprüfung die Temperierungsanforderung auftritt, wobei die Temperierungsanforderung den Schwellenwert überschreitet, wird die Dichtigkeitsprüfung bei typischen Ausführungsformen, wie auch bei dem Beispiel der Fig. 4, abgebrochen. Typischerweise ist vorgesehen, dass nach Abbruch einer Dichtigkeitsprüfung diese nachgeholt wird, beispielsweise sobald die Temperierungsanforderung den Schwellenwert wieder unterschreitet. Auf diese Weise kann einer Temperierung des Patienten Vorrang vor der Dichtigkeitsprüfung eingeräumt werden.

## Patentansprüche

1. Hypo-/Hyperthermiegerät (1) zur Verbindung mit einer Apparatur, insbesondere einem Oxygenator oder einem Plegiekreislauf, zur Ausbildung eines Temperierkreislaufs zur Temperierung von Blut, umfassend:
- eine Vorlaufleitung (20) mit einem Vorlaufanschluss (22) zum Anschluss an die Apparatur und einem in der Vorlaufleitung (20) angeordneten Vorlaufventil (21);
- eine Rücklaufleitung (30) mit einem Rücklaufanschluss (32) zum Anschluss an die Apparatur und einem in der Rücklaufleitung (30) angeordneten Rücklaufventil (31);
- eine Pumpe (4), welche zwischen der Rücklaufleitung (30) und der Vorlaufleitung (20) zur Förderung eines flüssigen Temperiermediums in einer Förderrichtung von der Rücklaufleitung (30) zu der Vorlaufleitung (20) angeordnet ist; und
- ein Druckmessgerät (6) zum Messen eines Drucks in der Vorlaufleitung (20) oder in der Rücklaufleitung (30).

2. Hypo-/Hyperthermiegerät (1) nach Anspruch 1, wobei das Druckmessgerät (6) in der Vorlaufleitung (20) zwischen dem Vorlaufventil (21) und dem Vorlaufanschluss (22) oder in der Rücklaufleitung (30) zwischen dem Rücklaufanschluss (32) und dem Rücklaufventil (31) angeordnet ist.

3. Hypo-/Hyperthermiegerät (1) nach Anspruch 1 oder 2 mit einer Kontrolleinheit, welche mit dem Vorlaufventil (21), dem Rücklaufventil (31) und dem Druckmessgerät (6) verbunden ist, und welche eingerichtet ist, um eine Dichtigkeitsprüfung durchzuführen, umfassend:
- Schließen des Rücklaufventils (31);
- Betreiben der Pumpe (4), um zwischen der Pumpe (4) und dem Rücklaufventil (31) einen Überdruck aufzubauen;
- Schließen des Vorlaufventils (21); und
- Messen eines ersten Kontroll-Drucks zwischen dem Vorlaufventil (21) und dem Rücklaufventil (31) mittels des Druckmessgeräts (6).

4. Hypo-/Hyperthermiegerät (1) nach Anspruch 3, wobei die Kontrolleinheit dazu konfiguriert ist, das Vorlaufventil (21) erst dann zu schließen, wenn von dem Druckmessgerät (6) ein zumindest im Wesentlichen konstanter Druck gemessen wird.

5. Hypo-/Hyperthermiegerät (1) nach einem der Ansprüche 3 oder 4, wobei die Kontrolleinheit ferner dazu konfiguriert ist, einen zweiten Kontroll-Druck in einem vorbestimmten Zeitabstand nach dem ersten Kontroll-Druck zu messen und eine Warnmitteilung auszugeben, falls eine zwischen dem ersten Kontroll-Druck und dem zweiten Kontroll-Druck auftretende Druckänderung oberhalb eines definierten Grenzwertes liegt.

6. Hypo-/Hyperthermiegerät (1) nach einem der Ansprüche 3 bis 5, wobei die Kontrolleinheit ferner konfiguriert ist zum automatischen Initiieren der Dichtigkeitsprüfung.

7. Hypo-/Hyperthermiegerät (1) nach einem der Ansprüche 3 bis 6, wobei die Kontrolleinheit ferner konfiguriert ist, die Dichtigkeitsprüfung in vorbestimmten Zeitintervallen durchzuführen.

8. Hypo-/Hyperthermiegerät (1) nach einem der Ansprüche 3 bis 7, wobei die Kontrolleinheit ferner konfiguriert ist, vor einem Durchführen der Dichtigkeitsprüfung Folgendes durchzuführen:
- Abfragen einer Temperierungsanforderung und Vergleichen der Temperierungsanforderung mit einem Schwellenwert; und
- Durchführen der Dichtigkeitsprüfung nur dann, falls die Temperierungsanforderung den Schwellenwert nicht überschreitet; und
- insbesondere bei Durchführen der Dichtigkeitsprüfung, Abbrechen der Dichtigkeitsprüfung falls während der Dichtigkeitsprüfung die Temperierungsanforderung den Schwellenwert überschreitet.

9. Hypo-/Hyperthermiegerät (1) nach einem der vorhergehenden Ansprüche, wobei die Pumpe eingerichtet ist, um einen maximalen Volumenstrom von 10 l/min zu fördern und/oder wobei das Hypo-/Hyperthermiegerät (1) dazu geeignet und/oder dazu zugelassen ist, in der Temperierung von Patienten eingesetzt zu werden, und/oder wobei das Hypo-/Hyperthermiegerät (1) desinfizierbar ist und/oder umfassend eine redundant ausgelegte Temperaturregelung und/oder umfassend eine redundante Überhitzungs-Abschaltung, und/oder umfassend eine redundante Pumpenüberwachung bezüglich Leistung, Drehzahl und/oder Stromaufnahme.

10. Dichtigkeitsprüfverfahren für einen Temperierkreislauf für eine Apparatur zur Temperierung von Blut, insbesondere einem Oxygenator oder einem Plegiekreislauf, mittels eines Hypo-/Hyperthermiegeräts (1) nach einem der vorhergehenden Ansprüche, insbesondere mit Durchführung des Dichtigkeitsprüfverfahrens während einer akuten Temperierung von Blut eines Patienten.

11. Dichtigkeitsprüfverfahren nach Anspruch 10, umfassend eine Dichtigkeitsprüfung mit:
- Schließen des Rücklaufventils (31);
- Betreiben der Pumpe (4), um zwischen der Pumpe (4) und dem Rücklaufventil (31) einen Überdruck aufzubauen;
- Schließen des Vorlaufventils (21); und
- Messen eines ersten Kontroll-Drucks zwischen dem Vorlaufventil (21) und dem Rücklaufventil (31) mittels des Druckmessgeräts (6).

12. Dichtigkeitsprüfverfahren nach Anspruch 11, wobei das Vorlaufventil (21) erst dann geschlossen wird, wenn von dem Druckmessgerät (6) ein zumindest im Wesentlichen konstanter Druck gemessen wird.

13. Dichtigkeitsprüfverfahren nach Anspruch 11 oder 12, mit:
- Messen eines zweiten Kontroll-Drucks in einem vorbestimmten Zeitabstand nach dem ersten Kontroll-Druck; und
- Ausgeben einer Warnmitteilung, falls eine zwischen dem ersten Kontroll-Druck und dem zweiten Kontroll-Druck auftretende Druckänderung oberhalb eines definierten Grenzwertes liegt.

14. Dichtigkeitsprüfverfahren nach einem der Ansprüche 11 bis 13, wobei die die Dichtigkeitsprüfung in vorbestimmten Zeitintervallen wiederholt durchgeführt wird.

15. Dichtigkeitsprüfverfahren nach einem der Ansprüche 11 bis 14, wobei vor einem Durchführen der Dichtigkeitsprüfung Folgendes durchgeführt wird:
- Abfragen einer Temperierungsanforderung und Vergleichen der Temperierungsanforderung mit einem Schwellenwert; und
- Durchführen der Dichtigkeitsprüfung nur dann, falls die Temperierungsanforderung den Schwellenwert nicht überschreitet; und
- Insbesondere bei Durchführen der Dichtigkeitsprüfung, Abbrechen der Dichtigkeitsprüfung falls während der Dichtigkeitsprüfung die Temperierungsanforderung den Schwellenwert überschreitet.
